# FASCICULE DE BREVET EUROPEEN

(11) **EP 1 023 435 B1**
(45) Date de publication et mention de la délivrance du brevet: **06.09.2006**
(21) Numéro de dépôt: 98954433.3
(22) Date de dépôt: 09.10.1998
(51) Int. Cl.: C12N 1/20, C12P 19/04, C08B 37/00, A61K 39/09, C07H 3/06

(54) **NOUVELLE ESPECE DE BACTERIE LACTIQUE**
NEUER LAKTOBAKTERIENSTAMM
NOVEL LACTIC ACID BACTERIA SPECIES

(30) Priorité: 17.10.1997 EP 97203245
(43) Date de publication de la demande: 02.08.2000
(73) Titulaire: SOCIETE DES PRODUITS NESTLE S.A., 1800 Vevey (CH)
(72) Inventeur: GAIER, Walter, F-14600 Honfleur (FR); PRIDMORE, David, CH-1000 Lausanne 26 (CH); STINGELE, Francesca, CH-1018 Lausanne (CH); NEESER, Jean-Richard, CH-1073 Savigny (CH); DESACHY, Patrice, CH-1009 Pully (CH); POT, Bruno, B-8200 Sint-Michiels Brugge (BE)
(74) Mandataire: Chautard, Cécile
(86) Numéro de dépôt international: PCT/EP1998/006636
(87) Numéro de publication internationale: WO 1999/020739

(56) Documents cités:
- WO-A-87/06267
- J.M.N. WILLERS ET AL.: "Immunochemical studies of type IV and two group-like (z) carbohydrate antigens of minute streptococci" ANTONIE VAN LEEUWENKOEK, vol. 39, 1973, pages 609-617, XP002101540
- M.R. WESSELS ET AL.: "Isolation and characterization of type IV group B Streptococcus capsular polysaccharide" INFECTION AND IMMUNITY, vol. 57, no. 4, 1989, pages 1089-1094, XP002101541

## Description

La présente invention a trait à une nouvelle espèce de bactérie lactique appartenant au genre *Streptococcus.*

### État de la technique

L'identification des bactéries lactiques est essentielle dans l'industrie laitière, et consiste à différencier entre plusieurs espèces des caractéristiques distinctives d'ordre morphologique, physiologique et/ou génétique.

Les caractères physiologiques distinctifs pour une espèce donnée de bactérie lactique peuvent être obtenus au moyen de différents tests incluant, par exemple, l'analyse de la capacité à fermenter différents sucres et l'analyse standardisée du profil de migration des protéines totales sur un gel d'électrophorèse du type SDS-PAGE (Pot *et al.,* Taxonomy of lactic acid bacteria, In Bacteriocins of lactic acid bacteria, Microbiology, Genetics and Applications, L. De Vuyst, and E. J. Vandamme ed., Blackie Academic & Professional, London, 1994).

Le profil de migration des protéines totales d'une espèce donnée, obtenu sur un gel d'électrophorèse SDS-PAGE, lorsqu'il est comparé à l'aide d'un densimètre à d'autres profils provenant d'autres espèces, permet de déterminer les relations taxonomiques entre les espèces. L'analyse numérique des différents profils, par exemple avec le logiciel GelCompar® permet notamment d'établir un degré de corrélation entre les espèces qui est fonction de différents paramètres, en particulier des algorithmes utilisés (GelCompar, version 4.0, Applied Maths, Kortrijk, Belgique; algorithmes: "Pearson Product Moment Correlation Coefficient, Unweighted pair Group Method using Average Linkage").

A ce jour, l'analyse comparative du profil des protéines totales par électrophorèse sur gel SDS-PAGE a fait ses preuves comme un moyen efficace pour distinguer des groupes homogènes et distincts d'espèces de bactéries lactiques (Pot *et al.,* Chemical Methods in Prokaryotic Systematics, Chapitre 14, M. Goodfellow A.G. O'Donnell Ed., John Wiley and Sons Ltd, 1994).

Avec cette méthode SDS-PAGE, les expériences précédentes ont ainsi montré que lorsque l'on obtient un degré de corrélation de Pearson de plus de 78 (sur une échelle de 100) entre deux souches de bactéries lactiques, on peut valablement en déduire que celles-ci appartiennent à la même espèce (Kersters *et al.,* Classification and Identification methods for lactic bacteria with emphasis on protein gel electrophoresis, In Acid Lactic Bacteria, Actes du Colloque Lactic'91, 33-40, Adria Normandie, France, 1992: Pot *et al.,* The potential role of a culture collection for identification and maintenance of lactic acid bacteria, Chapter 15, pp. 81-87, *In:* The Lactic Acid Bacteria, E. L. Foo, H. G. Griffin, R. Mollby, and C. G. Heden, Proceedings of the first lactic computer conference, Horizon Scientific Press, Norfolk).

A titre d'exemple, le groupe des bactéries lactiques acidophiles a pu être récemment divisé en 6 espèces distinctes au moyen de cette technique (Pot *et al.*, J. General Microb., 139, 513-517, 1993). De même, cette technique a pu récemment établir, en combinaison avec d'autres techniques, l'existence de plusieurs nouvelles espèces de *Streptococcus,* comme *Streptococcus dysgalactiae* subsp. *equisimilis, Streptococcus hyovaginalis* sp. *nov.* et *Streptococcus thoraltensis* sp. *nov* (Vandamme *et al.,* Int. J. Syst Bacteriol., 46, 774-781, 1996; Devriese *et al.,* Int. J. Syst Bacteriol, 1997, In press).

L'identification de nouvelles espèces de bactéries lactiques ne peut cependant se réduire à une analyse purement morphologique et/ou physiologique des bactéries. En effet, deux espèces très proches morphologiquement et/ou physiologiquement peuvent être éloignées d'un point de vue génétique. L'analyse de l'ARN ribosomique 16S des bactéries lactiques revêt ainsi une importance capitale pour déterminer définitivement l'appartenance d'une bactérie lactique à un genre ou une espèce déjà connue.

A ce jour, la "Deutsche Sammlung Von Mikroorganismen und Zellkulturen GmbH" (DSM, Braunschweig, Allemagne) a officiellement recensé environ 48 espèces différentes appartenant au genre *Streptococcus* (voir la liste ci-après). Toutes ces espèces possèdent un ARN ribosomique 16S qui est typique du genre *Streptococcus,* et peuvent être réparties dans des groupes distincts et homogènes, au moyen de la technique SDS-PAGE mentionnée ci-dessus.

La présente invention a trait à l'identification, au moyen des techniques d'identifications avancées ci-dessus, d'une nouvelle espèce de bactérie lactique appartenant au genre *Streptococcus,* et à son utilisation dans l'industrie laitière en général.

### Résumé de l'invention

A cet effet, l'invention concerne toute bactérie lactique,
- dont l'ARN ribosomique 16S est caractéristique du genre *Streptococcus* ayant une séquence nucléotidique SEQ iD NO:1 et,
- dont le profil en protéines totales, obtenu après culture de la bactérie dans un milieu MRS pendant 24 h à 28°C, extraction des protéines totales et migration des protéines sur un gel d'électrophorèse SDS-PAGE**,** présente un degré de corrélation de Pearson d'au moins 78 au regard du profil obtenu dans des conditions identiques avec la souche de bactérie lactique CNCM I**-**1920**,** mais distinct de ceux des espèces reconnues appartenant au genre *Streptococcus**,** à* savoir *S, acidominimus, S. agalactiae, S. alactolyticus, S anginosus, S bovis, S. canis, S. caprinus, S. constellatus, S cricetus, S. cristatus, S difficile, S. downei, S dysgalactiae ssp. dysgalactiae, S. dysgalactiae ssp. equisimilis**,** S. equi, S**.** equi ssp. equi S equi ssp. zooepidemicus, S equimus, S ferus, S gallolyticus, S gordonii S. hyointestinalis, S hyovaginalis, S. iniae, S intermedius, S. intestinalis, S. macacae, S. mitis, S. mutans, S. oralis, S. parasanguinis, S. parauberis, S. phocae, S. pleomorphus, S. pneumoniae, S. porcinus, S. pyogenes, S. ratti, S salivarius, S. sanguinis, S shiloi, S. sobrinus, S, suis, S. thermophilus, S. thoraltensis, S. uberis, S. vestibularis, S. viridans.*

L'invention concerne l'utilisation d'une souche de bactérie lactique selon l'invention pour la préparation d'une composition alimentaire, notamment un lait acidifié ou un fromage frais, par exemple.

L'invention concerne également l'utilisation d'un polysaccharide, susceptible d'être sécrété par la souche CNCM I-1924, constitué exclusivement par un enchaînement de glucose, galactose et N-acétylglucosamine dans une proportion respective de 3:2:1 pour la préparation d'une composition alimentaire ou pharmaceutique.

L'invention a pour objet une composition alimentaire ou pharmaceutique comprenant une souche de bactérie lactique selon l'invention.

Enfin, l'invention a aussi pour objet une composition alimentaire ou pharmaceutique comprenant un polysaccharide, susceptible d'être sécrété par la souche CNCM I-1924, constitué exclusivement par un enchaînement de glucose, galactose et N-acétylglucosamine dans une proportion moyenne respective de 3:2:1.

### Description détaillée de l'invention

L'appartenance de la nouvelle espèce selon l'invention au genre *Streptococcus* est de préférence mise en évidence en comparant la séquence nucléotidique de l'ARN ribosomique 16S des bactéries selon l'invention, ou de leur ADN génomique qui est transcrit en ARN ribosomique 16S, à celles des autres genres et espèces de bactéries lactiques connues à ce jour.

Plus particulièrement, on peut mettre en oeuvre la méthode exposée dans l'exemple 1 ci-après, ou encore d'autres méthodes connues par l'homme du métier (Schleifer et al., System, Appl. Microb., 18, 461-467, 1995; Ludwig et al., System. Appl. Microb., 15, 487-501, 1992), par exemple. La séquence nucléotidique SEQ ID NO:1 présentée dans la liste de séquence ci-après est caractéristique de cette nouvelle espèce, et présente des similitudes frappantes avec les séquences d'ARN ribosomique 16S trouvées dans les espèces de *Streptococcus* reconnues à ce jour.

La nouvelle espèce selon l'invention, qui constitue un nouveau groupe distinct et homogène, peut être aussi différenciée des autres espèces connues appartenant au genre *Streptococcus* au moyen de la technique d'identification des protéines totales sur un gel d'électrophorèse SDS-PAGE décrite plus haut

En particulier, cette nouvelle espèce donne un profil en protéines totales, obtenu après culture de la bactérie dans un milieu MRS pendant 24 h à 28°C, extraction des protéines totales et migration des protéines sur un gel d'électrophorèse SDS-PAGE, qui présente un degré de corrélation de Pearson d'au moins 78 (sur une échelle de 100) avec le profil obtenu dans des conditions identiques avec la souche de bactérie lactique CNCM I-1920, et cela au regard des profils obtenus dans des conditions identiques avec quelques-unes des différentes espèces de bactéries lactiques, notamment celles indiquées ci-après, par exemple.

Plus particulièrement, cette technique consiste à (1) isoler toutes les protéines (=protéines totales) d'une culture de bactérie lactique cultivée dans des conditions définies, (2) séparer les protéines par électrophorèse sur gel SDS-PAGE, (3) analyser la disposition des différentes fractions de protéines séparées à l'aide d'un densimètre qui mesure l'intensité et l'emplacement de chaque bande, (4) et à comparer le profil de protéines ainsi obtenu à ceux de plusieurs autres espèces de *Streptococcus* qui ont été, en parallèle ou au préalable, obtenus exactement dans les mêmes conditions opératoires.

Les techniques de préparation d'un profil de protéines totales telles que décrites ci-dessus, ainsi que l'analyse numérique de tels profils, sont bien connues par l'homme du métier. Néanmoins, les résultats ne sont fiables que dans la mesure où chaque étape du procédé est suffisamment standardisée. Face à ce besoin, des procédures standardisées sont ainsi régulièrement mises à la disposition du public par leurs auteurs. On peut citer notamment celle de Pot *et al.* présentée au cours d'un "workshop" organisé pas l'Union Européenne, à l'université de Ghent, en Belgique, le 12 au 16 septembre 1994 (Fingerprinting techniques for classification and identification of bacteria, SDS-PAGE of whole cell protein).

Le logiciel utilisé dans la technique d'identification sur gel d'électrophorèse SDS-PAGE revêt une importance déterminante dans la mesure où le degré de corrélation entre les espèces dépend des paramètres et des algorithmes utilisés par ce logiciel. Sans vouloir rentrer dans les détails théoriques, la comparaison quantitative de bandes captées par un densimètre et normalisées par un calculateur est faite de préférence avec le coefficient de corrélation de Pearson. La matrice de similitude ainsi obtenue peut être organisée à l'aide de l'algorithme UPGMA (unweighted pair group method using average linkage), un algorithme qui permet non seulement de regrouper les profils les plus similaires, mais aussi de construire des dendogrammes (voir Kersters K., Numerical methods in the classification and identification of bacteria by electrophoresis, *In* Computer-assisted Bacterial Systematics, 337-368, M. Goodfellow A.G. O'Donnell Ed., John Wiley and Sons Ltd, 1985).

De préférence, les souches de la nouvelle espèce présentent un profil en protéines totales ayant un degré de corrélation de Pearson d'au moins 85 au regard d'une des souches de bactéries de la nouvelle espèce. Pour les biotypes mentionnés ci-dessous, ce degré de corrélation de Pearson peut même dépasser 90, par exemple.

Au moyen de cette technique d'identification sur gel d'électrophorèse SDS-PAGE, la nouvelle espèce appartenant au genre *Streptococcus* selon l'invention peut se distinguer de toutes les espèces de *Streptococcus* reconnues à ce jour, à savoir les espèces *S. acidominimus, S. agalactiae, S. alactolyticus, S. anginosus. S. bovis, S. canis, S. caprinus, S. constellatus, S. cricetus, S. cristatus, S. difficile, S. downei, S. dysgalactiae ssp. dysgalactiae, S. dysgalactiae ssp. equisimilis, S. equi, S. equi ssp. equi, S. equi ssp. zooepidemicus, S. equinus, S. ferus, S. gallolyticus, S. gordonii, S. hyointestinalis, S. hyovaginalis, S. iniae, S. intermedius, S. intestinalis, S. macacae, S. mitis, S. mutans, S. oratis, S. parasanguinis, S. parauberis, S. phocae, S. pleomorphus, S. pneumoniae, S. porcinus, S. pyogenes, S. ratti, S. salivarius, S. sanguinis, S. shiloi, S. sobrinus, S. suis, S. thermophilus, S. thoraltensis, S. uberis, S. vestibularis, S. viridans.*

La nouvelle espèce selon l'invention se distingue également par cette technique des bactéries lactiques qui avaient été précédemment classées par erreur dans le genre des *Streptococcus* comme *S. adjacens* (nouvelle classification = *Abiotrophia adiacens), S. casseliflavus (=Enterococcus casseliflavus), S. cecorum (=Enterococcus cecorum), S. cremoris (=Lactococcus lactis subsp. cremoris), S. defectivus (=Abiotrophia defectiva), S. faecalis (=Enterococcus faecalis), S. faecium (=Enterococcus faecium), S. gallinarum (=Enterococcus gallinarum), S. garvieae (=Lactococcus garvieae), S. hansenii (=Ruminococcus hansenii), S. lactis (=Lactococcus lactis subsp. lactis), S. lactis cremoris (=Lactococcus lactis subsp. cremoris), S. lactis diacetilactis (=Lactococcus lactis subsp. lactis), S. morbillorum (=Gemella morbillorum), S. parvulus (=Atopobium parvulum), S. plantarum (=Lactococcus plantarum), S. raffinolactis (=Lactococcus raffinolactis)* et *S. saccharolyticus (=Enterococcus saccharolyticus*).

Les bactéries lactiques selon l'invention ont une morphologie caractéristique des *Lactococcus lactis,* par exemple, c'est à dire ont la forme de coques assemblés en chaînes.

Les sucres fermentescibles par la nouvelle espèce sont généralement au moins l'un des suivants, à savoir, le D-galactose, le D-glucose, le D-fructose, le D-mannose, la N-acétyl-(D-)glucosamine, la salicine, le cellobiose, le maltose, le lactose, le saccharose et le raffinose.

Parmi toutes les souches de la nouvelle espèce qui ont été isolées dans des laiteries en Suisse, 7 ont été déposées sous le traité de Budapest, à titre d'exemple, à la Collection Nationale de Culture de Microorganismes (CNCM), 25 rue du docteur Roux, 75724 Paris, le 14 octobre 1997, où elles se sont vues attribuer les numéros de dépôt CNCM I-1920, 1-1921, I-1922, I-I923, I-1924, I-1925 et I-1926.

Les souches de la nouvelle espèce peuvent être utilisées pour préparer un produit alimentaire ou pharmaceutique, notamment sous forme d'une culture fraîche, concentrée ou séchée, par exemple.

Les produits à base de lait sont bien évidemment préférés dans le cadre de l'invention. Par lait, on entend cependant désigner, d'une part, un lait d'origine animale, tel que les laits de vache, de chèvre, de brebis, de buflesse, de zébue, de jument, d'ânesse, de chamelle, etc. Ce lait peut être un lait à l'état natif, un lait reconstitué, un lait écrémé, ou un lait additionné de composés nécessaires à la croissance des bactéries ou au traitement subséquent du lait fermenté, comme des matières grasses, de protéines d'un extrait de levure, de la peptone et/ou un surfactant, par exemple. Le terme lait s'applique également à ce que l'on appelle communément un lait végétal, c'est à dire un extrait de matières végétales traitées ou non, telles que les légumineuses (soja, pois chiche, lentille, ect...) ou des oléagineuses (colza, soja, sésame, coton, ect...), extrait qui contient des protéines en solution ou en suspension colloïdale, coagulables par action chimique, par fermentation acide et/ou par la chaleur. Enfin, le mot lait désigne aussi des mélanges de laits animaux et de laits végétaux.

Les produits pharmaceutiques peuvent être toutes sortes de produits destinés à être administrés oralement, voire même d'une manière topique, qui comprennent un support pharmaceutique acceptable sur, ou dans lequel, on adjoint une culture de la nouvelle espèce sous forme fraîche, concentrée ou séchée, par exemple. Ces produits pharmaceutiques peuvent se présenter sous forme d'une suspension ingestible, d'un gel, d'un diffuseur, d'une capsule, d'une gélule, d'un sirop, ou sous n'importe quelle autre forme galénique connue par l'homme du métier.

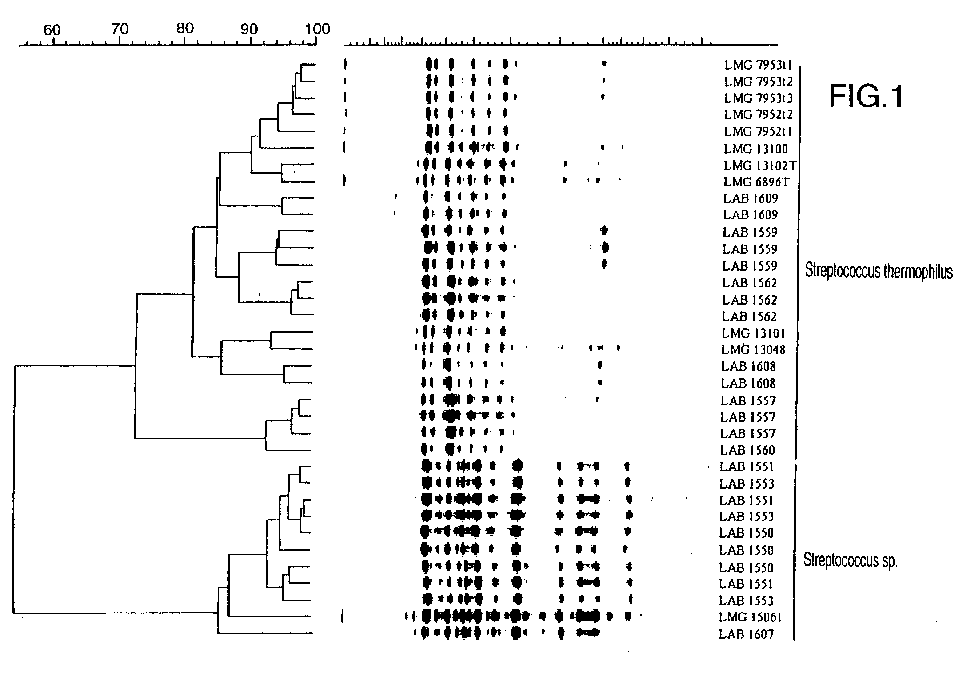

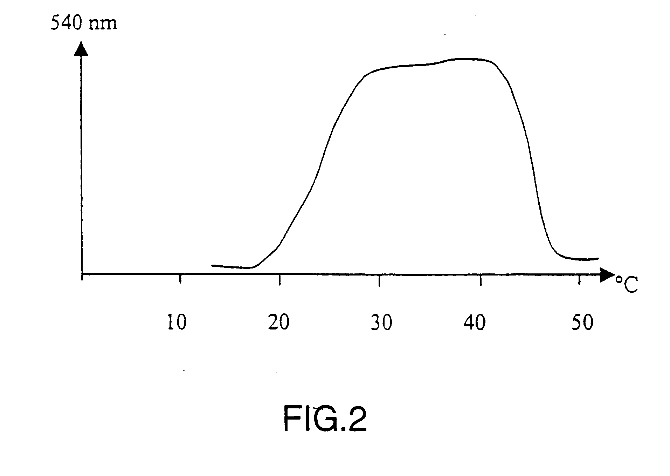

Par ailleurs, certaines souches de la nouvelle espèce selon l'invention, représentant un nouveau biotype de cette espèce, peuvent aussi présenter la propriété remarquable d'être à la fois mésophile et thermophile (biotype mésophile/thermophile). Les souches appartenant à ce biotype présentent en effet un optimum de croissance dès environ 28°C jusqu'environ 45°C. Cette propriété peut être facilement observée (1) en préparant plusieurs cultures d'un biotype mésophile/thermophile, en parallèle, à des températures s'échelonnant de 20 à 50°C, (2) en mesurant les absorbances des milieux après 16h de culture, par exemple, et (3) en réunissant les résultats sous forme d'un graphique représentant l'absorbance en fonction de la température (graditherme). La figure 1 est particulièrement représentative des graphiques que l'on peut obtenir avec ce biotype mésophile/thermophile selon l'invention. A titre d'indication, parmi les souches de la nouvelles espèces présentant ce biotype particulier, les souches CNCM 1-1920, I-1921 et I-1922 sont particulièrement représentatives, par exemple.

L'utilisation d'un biotype mésophile/thermophile dans l'industrie laitière revêt une importance non négligeable. En effet, on peut utiliser cette espèce pour la préparation de starters mésophiles ou thermophiles. On peut ainsi produire industriellement des lait acidifiés à 45°C pour obtenir un produit du type "yoghourt". On peut aussi produire industriellement des fromages frais en fermentant un lait en présence de présure à 28°C, et en séparant le caillé ainsi formé par centrifugation ou ultrafiltration. Les problèmes d'encrassement des machines liés à l'utilisation de ferments thermophiles sont ainsi éliminés (ces problèmes sont exposés dans la demande de brevet EP N°96203683.6)

Par ailleurs, d'autres souches de la nouvelle espèce selon l'invention, représentant un autre nouveau biotype de cette espèce, peuvent présenter la propriété remarquable de conférer une viscosité au milieu de fermentation (biotype texturant). Le caractère visqueux d'un lait fermenté par un biotype texturant selon l'invention peut être observé et déterminé comme décrit ci-après.
1. Par observation de la structure d'un lait acidifié par un biotype texturant en comparaison de celle du lait acidifié avec des cultures non-texturantes. Le lait non-visqueux adhère aux parois d'un gobelet en verre, tandis que le lait visqueux est cohérent sur lui-même.
2. Un autre essai peut être effectué à la pipette. La pipette est trempée dans le lait acidifié qui est aspiré en quantité d'environ 2 ml, puis la pipette est retirée du lait. Le lait visqueux forme un fil entre la pipette et la surface liquide, tandis que le lait non-visqueux ne donne pas lieu à ce phénomène. Lorsque le liquide est relâché de la pipette, le lait non-visqueux forme des gouttes distinctes tout comme l'eau, alors que le lait visqueux forme des gouttes se terminant par de longs fils qui aboutissent au bec de la pipette.
3. Lorsqu'un tube à essai rempli à peu près jusqu'au tiers de sa hauteur est agité à l'aide d'un agitateur rotatif, le lait non-visqueux remonte sur la face intérieure de la paroi, alors que l'ascension du lait visqueux est pratiquement nulle.

Le caractère visqueux de ce biotype particulier peut encore être déterminé à l'aide d'un paramètre rhéologique mesurant la viscosité. Quelques appareils commerciaux sont à même de déterminer ce paramètre, comme le rhéomètre Bohlin VOR (Bohlin GmbH, Allemagne). Conformément aux instructions du fournisseur, l'échantillon est placé entre un plateau et un cône tronqué de même diamètre (30 mm, angle de 5,4°, entrefer de 0,1 mm), puis l'échantillon est soumis à un gradient de vitesse de cisaillement rotatif continu qui le force à s'écouler. L'échantillon, en résistant contre la déformation, développe une force tangentielle appelée contrainte de cisaillement (shear stress). Cette contrainte qui est proportionnelle à la résistance à l'écoulement est mesurée par l'intermédiaire d'une barre de torsion. La viscosité de l'échantillon est alors déterminée, pour une vitesse de cisaillement donnée, par le rapport entre la contrainte de cisaillement (Pa) et la vitesse de cisaillement (s⁻¹) (voir aussi "Le Technoscope de Biofutur", Mai 97).

Les essais de mesure rhéologique du caractère texturant de ce biotype ont conduit à la définition suivante. Une bactérie lactique appartenant au biotype texturant selon l'invention est une bactérie qui, lorsqu'elle fermente un lait semi-écrémé à 38°C jusqu'à pH 5,2, donne une viscosité au milieu qui est supérieure à 100 mPa.s à une vitesse de cisaillement de l'ordre de 293 s⁻¹, par exemple. A titre d'indication, les souches CNCM I-1922, 1-1923, 1-1924, 1-1925 et 1-1926 sont particulièrement représentatives de ce biotype texturant, par exemple.

Ce biotype texturant revêt également une importance non négligeable dans l'industrie laitière, car sa capacité à donner une viscosité à un produit laitier est exceptionnellement élevée lorsqu'on la compare avec celles d'autres espèces de bactéries lactiques texturantes, en particulier avec les souches *Lactobacillus helveticus* CNCM I-1449, *Streptococcus thermophilus* CNCM I-1351, *Streptococcus thermophilus* CNCM I-1879, *Streptococcus thermophilus* CNCM I-1590, *Lactobacillus bulgaricus* CNCM 1-800 et *Leuconostoc mesenteroides ssp. cremoris* CNCM I-1692, dont il est fait mention respectivement dans les demandes de brevet EP699689, EP638642, EP97111379.0, EP750043, EP367918 et EP97201628.1.

On peut aussi remarquer que la production d'une viscosité peut s'effectuer, pour certaines souche, dans une gamme de température très large qui s'étend des températures mésophiles (25-30°C) aux températures termophiles (40-45°C). Cette particularité représente un avantage technologique évident.

Par ailleurs, certaines souches appartenant à ce nouveau biotype texturant produisent de surcroît un exopolysaccharide (EPS) de haut poids moléculaire, dont la composition en sucre est similaire à celle retrouvée dans les oligosaccharides du lait maternel humain. Cet EPS est en fait constitué par un enchaînement de glucose, galactose et N-acétylglucosamine dans une proportion de 3:2:1 respectivement (A. Kobata, *In* the Glycoconjugates, vol. 1, "Milk glycoproteins and oligosaccharides", p.423-440, Ed. I. Horowitz and W. Pigman, Ac. Press, N.Y., 1977). A titre d'indication, les souches CNCM I-1923, I-1924, I-1925 et I-1926 produisent ce polysaccharide.

Cet exopolysaccharide, sous forme native ou hydrolysée, pourrait ainsi satisfaire avantageusement une diète infantile complète.

Pour cela, on peut préparer une diète pour des enfants et/ou des nourrissons comprenant un lait qui a été acidifié avec au moins une souche de bactérie lactique productrice d'un EPS constitué par un enchaînement de glucose, galactose et N-acétylglucosamine dans une proportion de 3:2:1, respectivement, notamment avec les souches CNCM I-1924, I-1925 ou I-1926, par exemple.

On peut aussi préalablement isoler cet EPS d'un milieu de culture de ce biotype, et l'utiliser, sous forme native ou hydrolysée, comme ingrédient dans une diète infantile, par exemple.

L'isolation de l'EPS consiste généralement à retirer les protéines et les bactéries du milieu de culture et à isoler une fraction purifiée de l'EPS. On peut opérer l'extraction des protéines et des bactéries par précipitation avec un alcool ou l'acide trichloroacétique suivie d'une centrifugation, tandis que l'on peut purifier l'EPS par précipitation dans un solvant comme l'acétone suivie d'une centrifugation, par exemple. Si nécessaire, l'EPS peut être encore purifié au moyen d'une chromatographie par gel-filtration ou par affinité, par exemple.

Dans le contexte de la présente invention, l'isolation d'un EPS couvre également toutes les méthodes de production d'un EPS par fermentation suivie d'une concentration du milieu de culture par séchage ou ultrafiltration, par exemple. La concentration peut être opérée par toutes les méthodes connues de l'homme du métier, et en particulier par les méthodes de lyophilisation ou de pulvérisation sous un flux d'air chaud, par exemple. A cet effet, les méthodes décrites dans US3985901, EP298605 et EP63438 sont incorporées par référence dans la description de la présente invention.

Dans la mesure où les oligosaccharides maternels sont de petites tailles, il peut être avantageux de préalablement réaliser une hydrolyse partielle de l'EPS selon l'invention. De préférence, les conditions d'hydrolyse sont choisies de sorte à obtenir des oligosaccharides ayant 3 à 10 unités de sucre, soit donc des oligosaccharides ayant un poids moléculaire de l'ordre de 600 à 2000 Dalton, par exemple.

Plus particulièrement, on peut hydrolyser l'EPS selon l'invention dans une solution de trifluor acétique 0.5N (TFA) pendant 30-90 min et à 100°C, puis évaporer le TFA et récupérer les oligosaccharides.

Un produit infantil préféré comprend du matériel protéique hydrolysé de petit lait, à partir duquel des allergènes choisis dans un groupe d'allergènes consistant en l'α-lactalbumine, la β-lactoglobuline, la sérumalbumine et les immunoglobulines n'ont pas été éliminés et dans lequel le matériau protéique hydrolysé, y compris les allergènes hydrolysés, se présente sous forme de résidus d'hydrolyse ayant un poids moléculaire non supérieur à environ 10 000 Dalton, de sorte que le matériau hydrolysé est sensiblement exempt de protéines allergènes et d'allergènes d'origine protéique (= produit hypoallergénique conforme à la directive Européenne 96/4/EC; Fritsche *et al.,* Int. Arch. Aller and Appl. Imm., 93, 289-293, 1990).

On peut mélanger l'EPS selon l'invention, sous forme native ou partiellement hydrolysée, à ce matériel protéique hydrolysé de petit lait, et alors incorporer cette mixture, sous forme sèche ou non, dans de nombreuses préparations alimentaires à usage diététique, en particulier dans des aliments pour nourrissons ou dans des aliments facilement absorbables destinés en priorité aux personnes souffrant d'allergies, par exemple.

La présente invention est décrite plus en détail par les exemples présentés ci-après, qui sont précédés par une brève description des figures. Il va de soi, toutefois, que ces exemples sont donnés à titre d'illustration de l'objet de l'invention dont ils ne constituent en aucune manière une limitation. Les pourcentages sont donnés en poids sauf indication contraire.
- La figure 1 représente une photographie des profils de migration des protéines totales de plusieurs souches de la nouvelle espèce, sur gel d'électrophorèse SDS-PAGE, au regard de ceux obtenus avec des souches de *Streptococcus thermophilus.* Le degré de filiation des souches est indiqué à l'aide de l'échelle de corrélation de Pearson et au moyen de l'arborescence faisant face aux profils de protéines (les degrés de corrélation de Pearson de 55 à 100 sont représentés).
- La figure 2 représente le graditherme de la souche CNCM I-1920.

### Exemple 1 Identification d'une nouvelle espèce de Streptococcus

Plusieurs souches de bactéries lactiques isolées dans différentes laiteries en Suisse ont fait l'objet d'une identification génétique et physiologique comme suit. Les méthodes mises en oeuvres ainsi que les résultats obtenus, présentés ci-après, montrent que ces souches font partie d'un nouveau groupe de *Streptococcus,* qui est suffisamment distinct et homogène pour qu'on puisse le désigner comme regroupant une nouvelle espèce de bactérie lactique. A titre d'exemple, certaines souches appartenant à cette nouvelle espèce ont été déposées sous le traité de Budapest à la Collection Nationale de Culture de Microorganismes (CNCM), 25 rue due docteur Roux, 75724 Paris, le 14 octobre 1997, où elles ont reçu les numéros d'identification CNCM I-1920. I-1921, I-1922, I-1923, I-1924, I-1925 et I-1926.

1. Morphologie des souches isolées: on observe sous microscope une morphologie caractéristique des *Lactococcus lactis,* c'est à dire une forme de coques assemblées en chaînes.

2. Profil de fermentation des sucres des souches isolées: les sucres fermentescibles par les souches isolées sont généralement le D-galactose, le D-glucose, le D-fructose, le D-mannose, la N-acetyl-(D)glucosamine, la salicine, le cellobiose, le maltose, le lactose, le saccharose et le raffinose. Ce profil de fermentation est proche de celui obtenu avec l'espèce *Lactococcus lactis.*

### 3. ARN ribosomique 16S des souches isolées

On cultive les souches isolées dans 40 ml de milieu HJL à 37°C pendant 24 h, on récolte les bactéries par centrifugation, on remet en suspension chaque culot bactérien dans 2,5 ml de tampon TE (10 mM Tris pH8, 0,1 mM EDTA) contenant 10 mg/ml de lysozyme, et on incube le tout à 37°C pendant 1 h. On ajoute ensuite 100 µl d'une solution contenant 10 mg/ml de protéinase K, 250 µl d'une solution contenant 500 mM EDTA pH8.0, et 500 µl d'une solution contenant 10% de SDS. On incube le tout à 60°C pendant 1h de sorte à assurer une lyse complète des bactéries. Après avoir refroidit les mélanges, on leur ajoute 2,5 ml de phénol/chloroforme, on les centrifuge pendant 10 min dans une centrifugeuse Heraeus de sorte à séparer 2 phases, et on élimine la phase supérieure. On précipite l'ADN chromosomique présents dans la phase inférieure par addition de 2,5 ml d'une solution contenant 96% d'éthanol, et on agite doucement jusqu'à la formation d'un précipité. On retire l'ADN précipité à l'aide d'un cure-dent en bois, on les dépose dans un tube Eppendorf de 2 ml contenant 1 ml d'un tampon Tris (10 mM de Tris HCl pH 8,0, 10 mM d'EDTA et 10 µg/ml de RNase A), et on incube à 56°C pendant 1h. Après refroidissement, on extrait les différentes suspensions d'ADN avec 1 ml de phénol/chloroforme comme décrit ci-dessus, et on précipite les ADN chromosomiques avec de l'éthanol. Les ADN sont remis en suspension dans un tube Eppendorf contenant une quantité de tampon TE telle que la quantité finale d'ADN pour chaque souche isolée est de l'ordre de 250 µg/ml.

On amplifie par PCR un aliquot de 1 µl d'ADN de chaque souche isolée avec les amorces ayant les séquences nucléotidiques respectives SEQ ID NO:2 et SEQ ID NO:3 (voir la liste de séquence ci-après), pendant 30 cycles (95°C/30 sec, 40°C/30 sec et 72°C/2 min) en ayant recours à la polymerase Pwo de Boehringer. On purifie les produits de PCR à l'aide du kit QIAGEN QIAquick, et on élue les produits dans 50 µl de tampon TE. On digère un échantillon de 20 µl de chaque produit avec les enzymes de restriction *Bam*HI et *Sal*I, on sépare les fragments de 1,6 kb sur un gel d'agarose (1%), on les purifie à l'aide du kit QIAGEN QIAquick, on les clone ensuite dans le vecteur *E. coli* pK19 (Pridmore R.D., Gene 56, 309-312, 1987) digéré préalablement par *Bam*HI and *Sal*I et déphosphorylé, et on transforme les cellules compétentes *E. coli* strain BZ234 (collection de l'université de Bâle, Suisse) avec chaque produit de ligation. On sélectionne les transformants à 37°C sur milieu LB comprenant 50 µg/ml de kanamycine 30 ng/ml de X-gal et 10ng/ml d'IPTG. On cultive les colonies blanches contenant l'insert pendant 10 h sur milieu LB comprenant 50 µg/ml de kanamycine, et on isole les ADN plasmidiques à l'aide du kit QIAGEN QIAprep8.

On mélange 4 µl d'échantillon de chaque plasmide (1pmol/µl: provenant de chaque souche isolée) avec 4 µl d'amorces marquées IRD-41 (amorces de séquençage: MWG Biotech) et 17 µl d'H₂O. Pour chaque souche isolée, on dispense 4 aliquots de 6 µl dans 4 puits de 200 µl, et on leur ajoute 2 µl d'un mix de réaction (Amersham; RPN2536). On amplifie les mélanges par PCR dans le système Hybaid Omn-E par 1 cycle de 95°C pendant 2 min suivi par 25 cycles de 95°C/30 sec, 50°C/30 sec et 72°C/1 min. On sépare ensuite classiquement les produits de réaction sur gel de polyacrylamide, et on lit la séquence d'ADN pour chaque souche isolée. Les fragments d'ADN ainsi séquencés représentent la partie génomique de l'ARN ribosomique 16S.

Les résultats montrent que toutes les souches isolées contiennent une séquence nucléotidique similaire, voire identique à la séquence SEQ ID NO:1 qui est exposée dans la liste de séquence ci-après. Ces séquences présentent de nombreuses homologies avec les séquences d'ARN 16S trouvées dans les espèces de bactéries lactiques appartenant au genre *Streptococcus,* ce qui conduit à classer ces souches parmi le genre *Streptococcus.*

### 4. Identification par gel d'électrophorèse SDS-PAGE

Les essais ont été menés conformément aux instructions fournies par Pot *et al.* présentées au cours d'un "workshop" organisé par l'Union Européenne, à l'université de Ghent, en Belgique, le 12 au 16 septembre 1994 (Fingerprinting techniques for classification and identification of bacteria, SDS-PAGE of whole cell protein).

En résumé, pour cultiver les bactéries lactiques, on ensemence 10 ml de milieu MRS (de Man, Rogosa et Sharpe) avec une préculture MRS de chaque souche de la nouvelle espèce de bactérie lactique, ainsi que de chaque souche de référence couvrant le plus d'espèces possible de *Streptococcus.* On incube les milieux pendant 24h à 28°C, on les étale sur boîte de Petri comprenant un milieu frais MRS-agar, et on incube les boîtes pendant 24h à 28°C.

Pour préparer l'extrait renfermant les protéines des bactéries, on recouvre le milieu MRS-agar d'un tampon pH7,3 renfermant 0,008M de Na₂HPO₄.12H₂O, 0,002M de Na₂HPO₄.2H₂O et 8% de NaCl. On récupère les bactéries en grattant la surface du milieu gélifié, on filtre la suspension au travers d'une gaze Nylon, on la centrifuge 10 min à 9000rpm avec un rotor GSA, on récupère le culot que l'on reprend dans 1 ml de tampon précédent. On lave le culot en répétant la procédure de centrifugation-lavage, on récupère finalement environ 50mg de cellules auxquelles on ajoute un volume de tampon STB pH 6.8 (pour 1000 ml: 0.75 g Tris, 5ml C₂H₆OS, 5g de glycérol), on casse les cellules par des ultrasons (Labsonic 2000), on centrifuge les débris cellulaires, et on conserve le surnageant renfermant les protéines totales.

On prépare ensuite classiquement un gel de polyacrylamide SDS-PAGE de 1,5 mm d'épaisseur (Biorad-Protean ou Hoefer SE600) réticulé avec 12% d'acrylamide dans le cas du gel de séparation (12,6 cm de hauteur) et 5% d'acrylamide dans le cas du gel de concentration (1,4 cm de hauteur). Pour cela, on réalise notamment la polymérisation des deux parties de gel dans un bain thermostaté à 19°C pendant 24h et 1h respectivement, de sorte à réduire au maximum les imperfections du gel et à maximiser la reproductibilité des essais.

On sépare alors les protéines de chaque extrait sur le gel d'électrophorèse SDS-PAGE. Pour cela, on applique 6 mA pour chaque plaque contenant 20 pistes jusqu'à ce que le colorant atteigne 9,5 cm de distance depuis le haut du gel de séparation. On fixe ensuite les protéines dans le gel, on les colore, on sèche le gel sur un cellophane, on numérise le gel au moyen d'un densimètre (LKB Ultroscan Laser Densimètre, Suède) relié à un calculateur, et on compare les profils entre eux au moyen du logiciel GelCompar®, version 4.0, Applied Maths, Kortrijk, Belgique. Dans la mesure où les essais étaient suffisamment bien standardisés, les profils des différentes espèces de *Streptococcus* contenus dans une banque de données ont aussi été utilisés au cours de la comparaison numérique.

Les résultats montrent alors que toutes les souches testées appartenant à la nouvelle espèce se distinguent de toutes les espèces suivantes: *S*. *acidominimus, S. adjacens, S. agalactiae, S. alactolyticus. S. anginosus, S. bovis, S. canis, S. caprinus, S. casseliflavus, S. cecorum. S. constellatus, S. cremoris, S. cricetus, S. cristatus, S. defectivus, S. difficile, S. downei, S. dysgalactiae ssp. dysgalactiae, S. dysgalactiae ssp. equisimilis, S. equi, S. equi ssp. equi, S. equi ssp. zooepidemicus. S. equinus, S. faecalis, S. faecium, S. ferus, S. gallinarum, S. gallolyticus, S. garvieae, S. gordonii, S. hansenii, S. hyointestinalis, S. hyovaginalis. S. iniae, S. intermedius, S. intestinalis, S. lactis, S. lactis cremoris, S. lactis diacetilactis, S. macacae. S. mitis, S. morbillorum, S. mutans, S. oralis, S. parasanguinis, S. parauberis. S. parvulus, S. phocae, S. plantarum, S. pleomorphus, S. pneumoniae, S. porcinus, S. pyogenes, S. raffinolactis, S. ratti, S. saccharolyticus, S. salivarius. S. sanguinis, S. shiloi, S. sobrinus, S. suis, S. thermophilus, S. thoraltensis, S. uberis, S. vestibularis* et *S. viridans.*

L'ensemble des résultats montrent que degré de corrélation de Pearson entre les souches déposées est d'au moins 85. A titre d'indication, la figure 1 représente une photographie d'un des gels d'électrophorèse, la filiation sous forme d'une arborescence, ainsi que le degré de corrélation de Pearson (indiqué dans l'échelle supérieure gauche). Les souches LAB 1550, LAB 1551 et LAB 1553 font spécifiquement références aux souches CNCM I-1921, I-1922 et 1-1925. Les souches LMG15061 et LAB 1607 n'ont pas été déposées auprès de la CNCM, mais font manifestment parties de cette nouvelle espèce.

En définitive, toutes les souches isolées font clairement parties d'un groupe homogène, qui est distinct des autres espèces appartenant au genre *Streptococcus.*

### Exemple-2 Biotype mésophile/thermophile

Certaines souches isolées à l'exemple 1 représentent un nouveau biotype particulier dans la mesure où elles présentent la propriété remarquable d'être à la fois mésophile et thermophile.

Cette propriété est facilement observée (1) en préparant, en parallèle, plusieurs cultures d'un biotype mésophile/thermophile dans un milieu M17-lactose à des températures s'échelonnant de 20 à 50°C, (2) en mesurant les absorbances des milieux à 540 nm après 16h de culture, et (3) en réunissant les résultats sous forme d'un graphique représentant l'absorbance en fonction de la température (graditherme).

La figure 1 représente le graditherme obtenu avec la souche CNCM I-1920. Toutes les autres souches isolées appartenant à ce biotype particulier, notamment les souches CNCM 1-1921 et 1-1922, donnent également des gradithermes comparables.

### Exemple 3 Biotype texturant

Plusieurs souches isolées à l'exemple 1 présentent la propriété remarquable d'être extrêmement texturantes. Cette propriété peut être observée à l'aide du paramètre rhéologique de viscosité mesuré avec le rhéomètre rotatif Bohlin VOR (Bohlin GmbH, Allemagne).

Pour cela, on cultive certaines des souches isolées dans un lait semi-écrémé à 38°C jusqu'à un pH de 5,2. Conformément aux instructions du fournisseur, un échantillon de chaique milieu de culture est ensuite placé entre un plateau et un cône tronqué de même diamètre (30 mm, angle de 5,4°, entrefer de 0,1 mm), puis l'échantillon est soumis à un gradient de vitesse de cisaillement rotatif continu qui le force à s'écouler. On détermine alors la viscosité de l'échantillon à la vitesse de cisaillement de 293 s⁻¹. Les résultats des tests de rhéologie effectués avec certaines des souches isolées montrent que les milieux de culture ainsi fermentés présentent une viscosité supérieure à 100 mPa.s, voir une viscosité dépassant 200 mPa.s dans le cas des souches CNCM I-1922, I-1923, I-1924, I-1925 et I-1926.

Pour comparaison, on obtient, dans les mêmes conditions opératoires, des viscosités de l'ordre de 54, 94, 104, 158 et 165 mPa.s avec respectivement les souches *Lactobacillus helveticus* CNCM 1-1449, *Streptococcus thermophilus* CNCM I-1351, *Streptococcus thermophilus* CNCM I-1879, *Streptococcus thermophilus* CNCM I-1590, *Lactobacillus bulgaricus* CNCM I-800 et *Leuconostoc mesenteroides ssp. cremoris* CNCM 1-1692, dont il est fait mention respectivement dans les demandes de brevet EP699689, EP638642, EP97111379.0, EP750043, EP367918 et EP97201628.1 (Les souches CNCM I-800 et I-1692 sont réputées être des souches très texturantes).

### Exemple 4 Nouvel exopolysaccharide

Certaines souches isolées à l'exemple 1, appartenant au biotype texturant, notamment les souches CNCM I-1923. 1-1924. 1-1925 et 1-1926, produisent un EPS de haut poids moléculaire dont la composition en sucre est similaire à celles retrouvées dans certains oligosaccharides du lait maternel humain. L'analyse des sucres composant ce polysaccharide est effectuée de la manière suivante.

On cultive les souches de la nouvelle espèce dans un lait écrémé reconstitué à 10%, sous agitation, pendant 24 h à 30°C, le pH étant maintenu à 5,5 par addition d'une solution de NaOH 2N. On élimine les cellules bactériennes et les protéines du milieu de culture au moyen d'une précipitation dans un volume égal d'une solution de 25% en poids d'acide trichloroacétique, suivie d'une centrifugation (10000g, 1h). On précipite les EPS par addition d'un volume équivalent d'acétone, suivie d'une décantation pendant 20h à 4°C. On récupère les EPS par centrifugation, on reprend le culot dans une solution 0,1 M NH₄HCO₃ pH7, et on dialyse la suspension contre de l'eau pendant 24 h. On élimine alors les matériaux insolubles par ultracentrifugation, et on lyophilise le rétentat contenant l'EPS purifié. La quantité d'EPS purifié, exprimé en mg d'équivalent glucose, est de l'ordre de 40 mg par litre de culture.

On détermine le poids moléculaire de l'EPS au moyen d'une chromatographie par gel-filtration à l'aide d'une colonne Superose-6 connectée à un système FPLC (Pharmacia), comme décrit par Stingele *et al.,* J. bacteriol., 178, 1680-1690, 1996. Les résultats montrent que toutes les souches CNCM I-1923. I-1924, I-1925 et 1-1926 produisent un EPS d'une taille supérieure à 2 x 10⁶ Da.

100 mg d'équivalent glucose de l'EPS purifié sont hydrolysés dans du TFA 4N à 125°C pendant 1 h, avant d'être dérivatisés et analysés par chromatographie GLC selon la méthode décrite par Neeser *et al.* (Anal. Biochem., 142, 58-67, 1984). Les résultats montrent que les souches produisent un EPS constitué de glucose, galactose et N-acétylglucosamine dans une proportion moyenne 3:2:1, respectivement.

### Exemple 5 Produit infantile

On prépare un petit-lait hydrolyse à 18% par la trypsine en suivant les recommendations de US5039532, on le sèche traditionellement par pulvérisation sous un flux d'air chaud, et on lui incorpore entre 0,1 et 10% de l'EPS purifié sec décrit à l'exemple 4. Ce produit peut être rapidement reconstitué dans de l'eau. Il convient particulièrement à une diète pour enfants ou nourrissons du fait de ses propriétés hypoallergénique et tolérogène au lait de vache, et qu'il est complet d'un point de vue de la composition glucidique.

### Exemple 6 Produit infantile

On hydrolyse l'EPS purifié sec de l'exemple 4 dans une solution de trifluor acétique 0.5N (TFA) pendant 30-90 min et à 100°C, on évapore le TFA, on met en suspension l'hydrolysat dans de l'eau et on sépare les oligosaccharides ayant 3 à 10 unités de sucre (600 à 2000 Dalton) par ultrafiltration.

On prépare un petit-lait hydrolysé à 18% par la trypsine en suivant les recommendations de US5039532, on le sèche traditionellement par pulvérisation sous un flux d'air chaud, et on lui incorpore entre 0,1 et 10% des oligosaccharides purifiés décrits ci-dessus. Ce produit peut être rapidement reconstitué dans de l'eau. Il convient particulièrement à une diète pour enfants ou nourrissons du fait de ses propriétés hypoallergénique et tolérogène au lait de vache, et qu'il est complet d'un point de vue de la composition glucidique.

### Exemple 7 Produit pharmaceutique

On prépare une composition pharmaceutique sous forme d'une capsule fabriquée à base de gélatine et d'eau, et qui contient 5 à 50 mg de l'EPS purifié de l'exemple 4 ou des oligosaccharides purifiés de l'exemple 6.

### Exemple 8 Produit pharmaceutique

On prépare des pastilles constituées par une culture de la souche CNCM I-1924 lyophilisée, puis compressée avec un agent liant adéquat. Ces pastilles sont particulièrement indiquées pour restaurer une flore intestinale en bactéries lactiques et pour satisfaire une diète équilibrée en glucides complexes essentiels.

## Revendications

1. Souche de bactérie lactique,
- dont l'ARN ribosomique 16S est caractéristique du genre Streptococcus, ayant une séquence nucléotidique SEQ ID NO: 1 et
- dont le profil en protéines totales, obtenu après culture de la bactérie dans un milieu MRS pendant 24 h à 28°C, extraction des protéines totales et migration des protéines sur un gel d'électrophorèse SDS-PAGE, présente un degré de corrélation de Pearson d'au moins 78 au regard du profil obtenu dans des conditions identiques avec la souche de bactérie lactique CNCM I-1920, mais distinct de ceux des espèces reconnues appartenant au genre *Streptococcus, à savoir S. acidominimus, S. agalactiae, S. alactolyticus, S. anginosus, S. bovis, S. canis, S. caprinus, S. constellatus, S. cricetus, S. cristatus, S. difficile, S. downei, S. dysgalactiae ssp. dysgalactiae, S. dysgalactiae ssp. equisimilis, S. equi, S. equi ssp. equi, S. equi ssp. zooepidemicus, S. equinus, S. ferus, S. gallolyticus, S. gordonii, S. hyointestinalis, S. hyovaginalis, S. iniae, S. intermedius, S. intestinalis, S. macacae, S. mitis, S. mutans, S. oralis, S. parasanguinis, S. parauberis, S. phocae, S. pleomorphus, S. pneumoniae, S. porcinus, S. pyogenes, S. ratti, S. salivarius, S. sanguinis, S. shiloi, S. sobrinus, S. suis, S. thermophilus, S. thoraltensis, S. uberis, S. vestibularis, S. viridans.*

2. Souche de bactérie lactique selon la revendication 1, capable de fermenter le D-galactose, le D-glucose, le D-fructose, le D-mannose, la N-acetyl-(D-) glucosamine, la salicine, le cellobiose, le maltose, le lactose, le saccharose et le raffinose.

3. Souche de bactérie lactique selon la revendication 1 choisie parmi les souches CNCM I-1920, I-1921, I-1922, I-1923, I-1924, I-1925, I-1926.

4. Souche de bactérie lactique selon la revendication 1, **caractérisée en ce qu'**elle présente un optimum de croissance dès environ 28°C jusqu'à environ 45°C.

5. Souche de bactérie lactique selon la revendication 1, qui, lorsqu'elle fermente un lait semi-écrémé à 38°C jusqu'à pH 5,2, donne une viscosité au milieu qui est supérieure à 100 mPa.s à une vitesse de cisaillement de l'ordre de 293 s-1.

6. Souche de bactérie lactique selon la revendication 1, **caractérisée en ce qu'**elle produit un exopolysaccharide constitué exclusivement par un enchaînement de glucose, galactose et N-acétylglucosamine dans une proportion moyenne de 3:2:1 respectivement.

7. Utilisation d'une souche de bactérie lactique selon l'une des revendications 1 à 6 pour la préparation d'une composition alimentaire, notamment d'un lait acidifié.

8. Utilisation d'un polysaccharide, susceptible d'être sécrété par la souche CNCMI-1924, constitué exclusivement par un enchainement de glucose, galactose et N-acétylglucosamine dans une proportion moyenne respective de 3 : 2 : 1, pour la préparation d'une composition alimentaire.

9. . Utilisation selon le revendication 8, pour la préparation d'une composition destinée à satisfaire une diète infantile complète.

10. Composition alimentaire ou pharmaceutique comprenant une souche de bactérie lactique selon l'une des revendications 1 à 6.

11. Composition alimentaire ou pharmaceutique comprenant un polysaccharide, susceptible d'être sécrété par la souche CNCM-1924, constitué exclusivement par un enchaînement de glucose, galactose et N-acétylglucosamine dans une proportion moyenne respective de 3:2:1.

12. Composition selon la revendication 11, **caractérisée en ce qu'**il s'agit d'une composition infantile.

13. Composition selon la revendication 12, **caractérisée en ce qu'**il s'agit d'une composition infantile hypoallergénique.

14. Polysaccharide, susceptible d'être sécrété par la souche CNCM I-1924, constitué exclusivement par un enchaînement de glucose, galactose et N-acétylglucosamine dans une proportion moyenne respective de 3:2:1.

## Claims

1. A strain of lactic bacteria,
- the 16S ribosomal RNA of which is characteristic of the genus *Streptococcus,* having a nucleotide sequence SEQ ID no. 1 and
- the total protein profile of which, obtained after culturing the bacterium in an MRS medium for 24 h at 28°C, extraction of the total proteins and migration of the proteins onto an SDS-PAGE electrophoresis gel, exhibits a degree of Pearson correlation of at least 78 relative to the profile obtained under identical conditions with the strain of lactic bacteria CNCM 1-1920, but distinct from those of the recognised species belonging to the genus *Streptococcus,* namely *S*. *acidominimus, S. agalactiae, S. alactolyticus, S. anginosus, S. bovis, S. canis, S. caprinus, S. constellatus, S. cricetus, S. cristatus, S. difficile, S. downei, S. dysgalactiae ssp. dysgalactiae, S. dysgalactiae ssp. equisimilis, S. equi, S. equi ssp. equi, S. equi ssp. zooepidemicus, S. equinus, S. ferus, S. gallolyticus, S. gordonii, S. hyointestinalis, S. hyovaginalis, S. iniae, S. intermedius, S. intestinalis, S. macacae, S. mitis, S. mutans, S. oralis, S. parasanguinis, S. parauberis, S. phocae, S. pleomorphus, S. pneumoniae, S. porcinus, S. pyogenes, S. ratti, S. salivarius, S. sanguinis, S. shiloi, S. sobrinus, S. suis, S. thermophilus, S. thoraltensis, S. uberis, S. vestibularis, S. viridans.*

2. A strain of lactic bacteria according to claim 1, capable of fermenting D-galactose, D-glucose, D-fructose, D-mannose, N-acetyl-(D-)glucosamine, salicin, cellobiose, maltose, lactose, sucrose and raffinose.

3. A strain of lactic bacteria according to claim 1 selected from among the strains CNCM 1-1920, 1-1921, 1-1922, 1-1923, 1-1924, 1-1925, 1-1926.

4. A strain of lactic bacteria according to claim 1, **characterised in that** it exhibits optimum growth from approximately 28°C up to approximately 45°C.

5. A strain of lactic bacteria according to claim 1, which, when it ferments a semi-skimmed milk at 38°C up to pH 5.2, gives rise to a viscosity of the medium which is greater than 100 mPa·s at a shear rate of the order of 293 s⁻¹.

6. A strain of lactic bacteria according to claim 1, **characterised in that** it produces an exopolysaccharide solely made up of a linkage of glucose, galactose and N-acetylglucosamine in an average proportion of 3:2:1 respectively.

7. Use of a strain of lactic bacteria according to any one of claims 1 to 6 for the preparation of a food composition, in particular an acidified milk.

8. Use of a polysaccharide, likely to be secreted by the strain CNCM 1-1924, solely made up of a linkage of glucose, galactose and N-acetylglucosamine in a respective average proportion of 3:2:1, for the preparation of a food composition.

9. Use according to claim 8, for the preparation of a composition intended to provide a complete infant diet.

10. A food or pharmaceutical composition comprising a strain of lactic bacteria according to any one of claims 1 to 6.

11. A food or pharmaceutical composition comprising a polysaccharide, likely to be secreted by the strain CNCM-1924, solely made up of a linkage of glucose, galactose and N-acetylglucosamine in a respective average proportion of 3:2:1.

12. A composition according to claim 11, **characterised in that** it is a composition for infants.

13. A composition according to claim 12, **characterised in that** it is a hypoallergenic composition for infants.

14. A polysaccharide, likely to be secreted by the strain CNCM 1-1924, solely made up of a linkage of glucose, galactose and N-acetylglucosamine in a respective average proportion of 3:2:1.

## Patentansprüche

1. Milchbakterienstamm,
- dessen ribosomale 16S RNA für die Gattung Streptococcus charakteristisch ist und die Nucleotidsequenz SEQ ID NO: 1 aufweist und
- dessen Gesamtproteinprofil, das nach Kultur der Bakterie in einem MRS-Medium während 24 h bei 28°C, Extraktion der gesamten Proteine und Laufenlassen der Proteine auf einem SDS-PAGE-Elektrophoresegel erhalten wird, einen Korrelationsgrad nach Pearson von mindestens 78 gegenüber dem Profil aufweist, das bei gleichen Bedingungen mit dem Milchsäurebakterienstamm CNCM 1-1920 erhalten wird, sich jedoch von denen der zur Gattung Streptococcus gehörenden bekannten Arten unterscheidet, und zwar der Arten *S. acidominimus, S. agalactiae, S. alactolyticus, S. anginosus, S. bovis, S. canis, S. caprinus, S. constellatus, S. cricetus, S. cristatus, S. difficile, S. downei, S. dysgalactiae* ssp. *dysgalactiae, S. dysgalactiae* ssp. *equisimilis, S. equi, S. equi* ssp. *equi, S. equi* ssp. *zooepidemicus, S. equinus, S. ferus, S. gallolyticus, S. gordonii, S. hyointestinalis, S. hyovaginalis, S. iniae, S. intermedius, S. intestinalis, S. macacae, S. mitis, S. mutans, S. oralis, S. parasanguinis, S. parauberis, S. phocae, S. pleomorphus, S. pneuomiae, S. porcinus, S. pyogenes, S. ratti, S. salivarius, S. sanguinis, S. shiloi, S. sobrinus, S. suis, S. thermophilus, S. thoraltensis, S. uberis, S. vestibularis, S. viridans.*

2. Milchsäurebakterienstamm nach Anspruch 1, der D-Galctose, D-Glucose, D-Fructose, D-Mannose, N-Acetyl-(D-)Glucosamin, Salicin, Cellobiose, Maltose, Lactose, Saccharose und Raffinose fermentieren kann.

3. Milchsäurebakterienstamm nach Anspruch 1, der aus den Stämmen CNCM 1-1920, 1-1921, 1-1922, 1-1923, 1-1924, 1-1925, I-1926 ausgewählt ist.

4. Milchsäurebakterienstamm nach Anspruch 1, **dadurch gekennzeichnet, dass** er etwa ab 28°C bis etwa 45°C ein Wachstumsoptimum aufweist.

5. Milchsäurebakterienstamm nach Anspruch 1, der, wenn er halbentrahmte Milch bei 38°C bis zu pH 5,2 fermentiert, dem Medium eine Viskosität verleiht, die bei einer Schergeschwindigkeit von etwa 293 s⁻¹ höher als 100 mPa.s ist.

6. Milchsäurebakterienstamm nach Anspruch 1, **dadurch gekennzeichnet, dass** er ein Exopolysaccharid erzeugt, das ausschließlich aus einer Verkettung von Glucose, Galactose und N-Acetylglucosamin in einem mittleren Verhältnis von 3:2:1 besteht.

7. Verwendung eines Milchsäurebakterienstamms nach einem der Ansprüche 1 bis 6 für die Herstellung einer Nahrungsmittelzusammensetzung, insbesondere angesäuerter Milch.

8. Verwendung von Polysaccharid, das von dem Stamm CNCM 1-1924 sekretiert werden kann und ausschließlich aus einer Verkettung von Glucose, Galactose und N-Acetylglucosamin in einem mittleren Verhältnis von 3:2:1 besteht, für die Herstellung einer Nahrungsmittelzusammensetzung.

9. Verwendung nach Anspruch 8 für die Herstellung einer Zusammensetzung, die dazu bestimmt ist, eine vollständige Kinderdiät zu bilden.

10. Nahrungsmittelzusammensetzung oder pharmazeutische Zusammensetzung, umfassend einen Milchsäurebakterienstamm nach einem der Ansprüche 1 bis 6.

11. Nahrungsmittelzusammensetzung oder pharmazeutische Zusammensetzung, umfassend ein Polysaccharid, das von dem Stamm CNCM-1924 sekretiert werden kann und ausschließlich aus einer Verkettung von Glucose, Galactose und N-Acetylglucosamin in einem mittleren Verhältnis von 3:2:1 besteht.

12. Zusammensetzung nach Anspruch 11, **dadurch gekennzeichnet, dass** es sich um eine Kindernahrungszusammensetzung handelt.

13. Zusammensetzung nach Anspruch 12, **dadurch gekennzeichnet, dass** es sich eine hypoallergene Kindernahrungszusammensetzung handelt.

14. Polysaccharid, das von dem Stamm CNCM-1924 sekretiert werden kann und ausschließlich aus einer Verkettung von Glucose, Galactose und N-Acetylglucosamin in einem mittleren Verhältnis von 3:2:1 besteht.


### LISTE DE SEQUENCES

(1) INFORMATIONS GENERALES:
   (i) DEPOSANT:
      (A) NOM: SOCIETE DES PRODUITS NESTLE
      (B) RUE: Av NESTLE 55
      (C) VILLE: VEVEY
      (D) ETAT OU PROVINCE: VAUD
      (E) PAYS: SUISSE
      (F) CODE POSTAL: 1500
   (ii) TITRE DE L' INVENTION: Nouvelle espèce de bacterie lactique
   (iii) NOMBRE DE SEQUENCES: 3
   (iv) FORME DECHIFFRABLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: Patent In Release #1.0, Version #1.30 (OEB)
(2) INFORMATIONS POUR LA SEQ ID NO: 1:
   (i) CARACTERISTIQUES DE LA SEQUENCE:
      (A) LONGUEUR: 1522 paires de bases
      (B) TYPE: nucl,otide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 1:
   GTCGACAGAG TTCGATCCTG GCTCAGGACG AACGCTGGCG GCGTGCCTAA TACATGCAAG     60
   TAGAACGCTG AAGACTTTAG CTTGCTAGAG TTGGAAGAGT TGCGAACGGG TGAGTAACGC    120
   GTAGGTAACC TGCCTATTAG TGGGGGATAA CTATTGGAAA CGATAGCTAA TACCGCATAA    180
   TAGTGTTTAA CACATGTTAG AGACTTAAAA GATGCAATTG CATCACTAGT AGATGGACCT    240
   GCGTTGTATT AGCTAGTTGG TGGGGTAACG GCCTACCAAG GCGACGATAC ATAGCCGACC    300
   TGAGAGGGTG ATCGGCCACA CTGGGACTGA GACACGGCCC AGACTCCTAC GGGAGGCAGC    360
   AGTAGGGAAT CTTCGGCAAT GGGGGCAACC TGACCGAGCA ACGCCGCGTG AGTGAAGAAG    420
   GTTTTCGGAT CGTAAAGCTC TGTTGTAAGA GAAGAACGTG TGTGAGAGTG GAAAGTTCAC    480
   ACAGTGACGG TAACTTACCA GAAAGGGACG GCTAACTACG TGCCAGCAGC CGCGGTAATA    540
   CGTAGGTCCC GAGCGTTGTC CGGATTTATT GGGCGTAAAG CGAGCGCAGG CGGTTTAATA    600
   AGTCTGAAGT TAAAGGCAGT GGCTTAACCA TTGTTCGCTT TGGAAACTGT TAAACTTGAG    660
   TGCAGAAGGG GAGAGTGGAA TTCCATGTGT AGCGGTGAAA TGCGTAGATA TATGGAGGAA    720
   CACCGGTGGC GAAAGCGGCT CTCTGGTCTG TAACTGACGC TGAGGCTCGA AAGCGTGGGG    780
   AGCAAACAGG ATTAGATACC CTGGTAGTCC ACGCCGTAAA CGATGAGTGC TAGGTGTTAG    840
   GCCCTTTCCG GGCTTAGTGC CCGCAGCTAA CGCATTAAGC ACTCCGCCTG GGGAGTACGA    900
   CCGCAAGGTT GAAACTCAAA GGAATTGACG GGGGCCGCAC AAGCGGTGGA GCATGTGGTT    960
   TAATTCGAAG CAACGCGAAG AACTTACCAG GTCTTGACAT CCCGATGCTA TTTCTAGAGA   1020
   TAGAAAGTTT CTTCGGAACA TCGGTGACAG GTGGTGCATG GTTGTCGTCA GCTCGTGTCG   1080
   TGAGATGTTG GGTTAAGTCC CGCAACGAGC GCAACCCCTA TTGTTAGTTG CCATCATTCA   1140
   GTTGGGCACT CTAGCGAGAC TGCCGGTGAT AAACCGGAGG AAGGTGGGGA TGACGTCAAA   1200
   TCATCATGCC CCTTATGACC TGGGCTACAC ACGTGCTACA ATGGTTGGTA CAACGAGTCG   1260
   CAAGCCGGTG ACGGCAAGCA AATCTCTTAA AGCCAATCTC AGTTCGGATT GTAGGCTGCA   1320
   ACTCGCCTAC ATGAAGTCGG AATCGCTAGT AATCGCGGAT CAGCACGCCG CGGTGAATAC   1380
   GTTCCCGGGC CTTGTACACA CCGCCCGTCA CACCACGAGA GTTTGTAACA CCCGAAGTCG   1440
   GTGAGGTAAC CTTTTAGGAN CCAGCCGCCT AAGGTGGGAC AGATGATTGG GGTGAAGTCG   1500
   TAACAAGGTA ACCGTAGGAT CC                                            1522
(2) INFORMATIONS POUR LA SEQ ID NO: 2:
   (i) CARACTERISTIQUES DE LA SEQUENCE:
      (A) LONGUEUR: 34 paires de bases
      (B) TYPE: nucl,otide
      (C) NOMBRE DE BRINS: simple
      (D) CONFIGURATION: linéaire
   (ii) TYPE DE MOLECULE: Autre acide nucl,ique
      (A) DESCRIPTION: /desc = "amorce"
   (xi) DESCRIPTION DE LA SEQUENCE: SEQ ID NO: 2:
      ATATCCGTTT TTTCGACAGA GTTYGATYCT GGCT 34
(2) INFORMATIONS POUR LA SEQ ID NO: 3:
   (i) CARACTERISTIQUES DE LA SEQUENCE:
      (A) LONGUEUR: 33 paires de bases
      (B) TYPE: nucl,otide
      (C) NOMBRE DE BRINS: simple
      (D) CONFIGURATION: lin,aire
   (ii) TYPE DE MOLECULE: Autre acide nucléique
      (A) DESCRIPTION: /desc = "amorce"
   (xi) DESCRIPTION DE LA SEQUENCE: SEQ ID NO: 3:
      ATATCCGGAT CCTACGGYTA CCTTGTTACG ACT 33